# EUROPÄISCHE PATENTSCHRIFT

(11) **EP 2 422 861 B1**
(45) Veröffentlichungstag und Bekanntmachung des Hinweises auf die Patenterteilung: **29.03.2023**
(21) Anmeldenummer: 10174689.9
(22) Anmeldetag: 31.08.2010
(51) Int. Cl.: B01D 15/38, C07K 1/22, C07K 14/415, B01J 20/32, B01J 20/286, G01N 30/00

(54) **Verfahren zur Identifizierung von zur Komplexbildung befähigten Naturstoffen**
Method for identifying natural substances able to form a complex
Méthode d'identification de substances naturelles aptes à former un complexe

(43) Veröffentlichungstag der Anmeldung: 29.02.2012
(73) Patentinhaber: Symrise AG, 37603 Holzminden (DE)
(72) Erfinder: Degenhardt, Andreas, 37603 Holzminden (DE); Krammer, Gerhard, 37603 Holzminden (DE); Schulze, Nicole, 31061 Alfeld (DE)
(74) Vertreter: Eisenführ Speiser

(56) Entgegenhaltungen:
- EP-A1- 1 504 677
- US-A1- 2008 145 950
- DUHITA N; HIWASA-TANASE K; YOSHIDA S; EZURA H: "Single-Step Purification of Native Miraculin Using Immobilized Metal-Affinity Chromatography" JOURNAL OF AGRICULTURAL AND FOOD CHEMISTRY, Bd. 57, Nr. 12, 24. Juni 2009 (2009-06-24) , Seiten 5148-5151, XP002605776 AMERCAN CHEMICAL SOCIETY ISSN: 0021-8561 DOI: 10.1021/jf9004065
- NUNES FERNANDO M; COIMBRA MANUEL A: "Melanoidins from Coffee Infusions. Fractionation, Chemical Characterization, and Effect of the Degree of Roast" JOURNAL OF AGRICULTURAL AND FOOD CHEMISTRY, Bd. 55, Nr. 10, 16. Mai 2007 (2007-05-16), Seiten 3967-3977, XP002605777 AMERICAN CHEMICAL SOCIETY ISSN: 0021-8561 DOI: 10.1021/jf063735h
- LIU Y ET AL: "Copper(II)-iminodiacetic acid chelating resin as a stationary phase in the immobilized metal ion affinity chromatography of some aromatic amines", JOURNAL OF CHROMATOGRAPHY A, ELSEVIER, AMSTERDAM, NL, vol. 515 31 August 1990 (1990-08-31), pages 169-173, XP026476245, ISSN: 0021-9673, DOI: 10.1016/S0021-9673(01)89310-9 [retrieved on 1990-08-31]
- YUN-XIAO ZHANG ET AL: "Purification of Arachidonic Acid from Fungal Single-Cell Oil via Al2O3-Supported CuSO4 Column Chromatography", JAOCS, JOURNAL OF THE AMERICAN OIL CHEMISTS' SOCIETY, vol. 83, no. 7, 1 January 2006 (2006-01-01), pages 659-662, XP055661876,

## Beschreibung

Die vorliegende Erfindung betrifft ein Verfahren zur Identifizierung eines Naturstoffes, der zur Komplexbildung mit Ni²⁺, Cu²⁺ und/oder Fe²⁺ - Ionen befähigt ist, wobei ein naturstoffhaltiger Extrakt über eine mit Ni²⁺, Cu²⁺ und/oder Fe²⁺ - Ionen beladene stationäre Phase, welche zur immobilisierten Metallionen-Affinitäts-Chromatographie (IMAC) geeignet ist, geleitet wird. Die Erfindung betrifft ferner die Verwendung einer mit Ni²⁺, Cu²⁺ und/oder Fe²⁺ - Ionen beladenen stationären Phase in einem solchen Verfahren.

Aromakompositionen (häufig auch als Aromen bezeichnet) enthalten zumindest zwei verschiedene sensorisch wirksame Stoffe. Zumeist sind Aromen jedoch komplexe Gemische einer Vielzahl sensorisch wirksamer Komponenten. Die sensorisch wirksamen Substanzen können dabei flüchtig (Geruchs- oder Aromastoffe) oder nichtflüchtig (Geschmackstoffe) sein. Die flüchtigen Aromastoffe können vom Menschen sowohl orthonasal als auch retronasal wahrgenommen werden. Die Geschmackstoffe interagieren mit den Geschmacksrezeptoren der Zunge und sind für die gustatorischen (geschmacklichen) Eindrücke süß, sauer, bitter, salzig und umami verantwortlich, daneben werden auch andere, oftmals trigeminale Reize, wie beispielsweise scharfe, brennende, kühlende, elektrisierende ("tingling") oder prickelnde Effekte, wahrgenommen.

Lebensmittel im Rahmen des vorliegenden Textes sind oral konsumierbare Zubereitungen, die dazu bestimmt sind, in die menschliche Mundhöhle eingebracht zu werden, dort eine bestimmte Zeit zu verbleiben und anschließend entweder verzehrt (z.B. verzehrfertige Lebensmittel, Getränke) oder wieder aus der Mundhöhle entfernt zu werden (z.B. Kaugummis).

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitgeschluckt zu werden, oder bei denen ein Verschlucken vorauszusehen ist. Auch bestimmte Produkte, die üblicherweise wieder aus der Mundhöhle entfernt werden (z.B. Kaugummis) sind im Rahmen des vorliegenden Textes als Lebensmittel zu verstehen.

Im Bereich der oral konsumierbare Zubereitungen, insbesondere im Bereich der Lebensmittel, stellt die Aroma- und Geschmacksstabilität ein wichtiges Qualitätskriterium dar. Oral konsumierbare Zubereitungen, insbesondere Lebensmittel, sollen über einen möglichst langen Zeitraum einen möglichst gleichbleibenden sensorischen Eindruck erzeugen, insbesondere ein möglichst gleichbleibendes Aroma- und Geschmacksprofil aufweisen.

Häufig können oxidative Abbaureaktionen bestimmter Bestandteile von oral konsumierbaren Zubereitungen, insbesondere von Lebensmitteln, dazu führen, dass sich das Aroma und/oder der Geschmack mit der Zeit verändert, es also zu sensorischer, insbesondere geruchlicher und/oder geschmacklicher, Instabilität kommt. Diese sensorisch unerwünschten Veränderungen werden als Aromafehler (Fehlnoten, "Off-Flavor") bezeichnet. Dabei können Spuren von Metallionen, insbesondere von Ni²⁺, Cu²⁺ und/oder Fe²⁺ - Ionen, merklich an den entsprechenden oxidativen Abbaureaktionen beteiligt sein.

In Lebensmitteln können flüchtige Verbindungen durch die Oxidation ungesättigter Substanzen entstehen, z.B. durch Lipidperoxidation, die sehr niedrige Geruchs- und Geschmacksschwellen aufweisen, so dass sie auch in geringer Konzentration unerwünschte Noten in den Aromaprofilen von Lebensmittels hervorrufen und Aromafehler verursachen können. Solche Instabilitäten und Aromafehler sind beispielsweise von Aroma und/oder Geschmack oxidationsempfindlicher Lebensmittel wie Bier, Dressings und Margarinen bekannt.

Bislang werden zur sensorischen Stabilisierung häufig Antioxidantien wie beispielsweise Ascorbinsäure (Vitamin C), Ascorbate, Tocopherole oder BHT eingesetzt. Diese Substanzen wirken antioxidativ oder reduzierend.

Ferner können oral konsumierbare Zubereitungen, insbesondere Lebensmittel, bestimmte Metallionen komplexierende Verbindungen zugesetzt werden, wie beispielweise Calcium-di-Natriumethylendiamintetraacetat (E-Nummer 385). EDTA und ETDA-Salze weisen jedoch eine zu stark Metallionen-komplexierende Wirkung auf und können dem menschlichen Körper unerwünschterweise Metallionen entziehen, was insbesondere im Falle von Fe²⁺ - Ionen nachteilig ist.

Es wäre daher wünschenswert vom Menschen oral konsumierbare Substanzen zu finden, die Metallionen, insbesondere Ni²⁺, Cu²⁺ und/oder Fe²⁺ - Ionen, komplexieren können und dabei diese Metallionen weniger stark komplexieren als EDTA (Ethylendiamintetraessigsäure) bzw. EDTA-Salze. Zudem wäre es von Vorteil, wenn diese Substanzen natürlichen Ursprungs, d.h. aus natürlichen Quellen erhältlich, wären. Die Aufgabe der vorliegenden Erfindung bestand darin, ein Verfahren zum Auffinden von vom Menschen verzehrbaren Naturstoffen anzugeben, wobei diese Naturstoffe zur Erhöhung der sensorischen Stabilität, insbesondere der geruchlichen und/oder geschmacklichen Stabilität, von oral konsumierbaren Zubereitungen, insbesondere von Lebensmitteln, geeignet sind.

Diese Naturstoffe sollten insbesondere die katalytische Aktivität eventuell vorhandener Metallionen und oxidative Abbaureaktionen reduzieren oder verhindern, die zur Beeinträchtigung der sensorischen Stabilität von oral konsumierbaren Zubereitungen führen.

Die vorliegende Erfindung betrifft daher ein Verfahren zur Identifizierung eines Naturstoffes, der zur Komplexbildung mit Ni²⁺, Cu²⁺ und/oder Fe²⁺ befähigt ist, gekennzeichnet durch folgende Schritte:
(a) Bereitstellen eines wässrigen, alkoholischen oder wässrig-alkoholischen naturstoffhaltigen Extraktes,
(b) Bereitstellen einer Vorrichtung, enthaltend eine mit Ni²⁺, Cu²⁺ und/oder Fe²⁺ - Ionen beladene stationäre Phase, welche zur immobilisierten Metallionen-Affinitäts-Chromatographie (IMAC) geeignet ist (IMAC-Vorrichtung), wobei der eingesetzte naturstoffhaltige Extrakt ein pflanzlicher Lebensmittelextrakt ist, der durch Extraktion eines oder mehrerer zum Verzehr durch den Menschen geeigneter Pflanzenteile erhalten wurde, ausgewählt aus der Gruppe bestehend aus Stängeln, Blättern, Blüten, Wurzeln, Samen, Früchten,
(c) Durchleiten des naturstoffhaltigen Extraktes aus Schritt (a) durch eine Vorrichtung aus Schritt (b), so dass eine Adsorption des zur Komplexbildung mit Ni²⁺, Cu²⁺ und/oder Fe²⁺ - Ionen befähigten Naturstoffes an der stationären Phase der IMAC-Vorrichtung erfolgt,
(d) gegebenenfalls Spülen der mit dem zur Komplexbildung mit Ni²⁺, Cu²⁺ und/oder Fe²⁺ - Ionen befähigten Naturstoff beladenen stationären Phase der IMAC-Vorrichtung aus Schritt (c) mit einem Lösungsmittel, ausgewählt aus der Gruppe bestehend aus reinem Wasser, vorzugsweise ionenfreiem Wasser, und Wasser-Ethanol-Gemischen,
(e) Desorption des Naturstoffes von der stationären Phase der IMAC-Vorrichtung aus Schritt (c) oder gegebenenfalls aus Schritt (d), bevorzugt mittels Durchleiten einer Lösung enthaltend ein oder mehrere Desorptionsmittel durch die IMAC-Vorrichtung aus Schritt (c) oder gegebenenfalls aus Schritt (d),
(f) gegebenenfalls Abtrennung des Naturstoffes vom Desorptionsmittel,
(g) Identifizierung des zur Komplexbildung mit Ni²⁺, Cu²⁺ und/oder Fe²⁺ - Ionen befähigten Naturstoffes,
wobei der Naturstoff kein Protein ist und ein Molekulargewicht von kleiner oder gleich 1000 g/mol aufweist..

Das erfindungsgemäße Verfahren ermöglicht es, aus komplex zusammengesetzten naturstoffhaltigen Extrakten selektiv diejenigen Naturstoffe, die zur Komplexbildung mit Ni²⁺, Cu²⁺ und/oder Fe²⁺ - Ionen befähigt sind, anzureichern und zu identifizieren.

Die Komplexbildung mit Fe²⁺ - Ionen ist im Rahmen der vorliegenden Erfindung besonders relevant, da Fe²⁺ - Ionen eine häufige Kontamination in Lebensmitteln aus der Natur darstellen.

Daneben sind Ni²⁺ - Ionen von Bedeutung, da diese als Spuren von Katalysatoren der Fetthydrierung in den Fetten verbleiben können und auf diese Weise in Lebensmittel gelangen können.

Der durch das erfindungsgemäße Verfahren identifizierte Naturstoff ist vorzugsweise ein zum Verzehr durch den Menschen geeigneter Naturstoff, insbesondere ein Aroma- oder Geschmackstoff.

Der in dem erfindungsgemäßen Verfahren in Schritt (a) bereitgestellte und in Schritt (c) eingesetzte wässrige, alkoholische oder wässrig-alkoholische naturstoffhaltige Extrakt ist meist ein komplexes Gemisch und enthält vorzugsweise
- 2, 3, 4, 5, 6, 7, 8, 9, 10 oder mehr verschiedene Aromastoffe, und/oder
- 2, 3, 4, 5 oder mehr verschiedene Geschmackstoffe.

Der in dem erfindungsgemäßen Verfahren in Schritt (a) bereitgestellte und in Schritt (c) eingesetzte naturstoffhaltige Extrakt ist beispielsweise ein Extrakt aus grünem Tee, Malz oder Kaffeebohnen.

WO 02/077024 offenbart ein Protein, welches eine Metallionen-Bindungsaktivität an Ni- und Cu-Ionen zeigt. Dieses Protein wurde aus der Auster *Crassostrea gigas* erhalten und hatte ein Molekulargewicht von etwa 20 kDa bzw. von etwa 31 kDa.

YUN-XIAO-ZHANG ET AL: "Purification of Arachidonic Acid from Fungal Single-Cell Oil via Al2O3-Supported CuSO4 Column Chromatography", JOURNAL OF THE AMERICAN OIL CHEMISTS' SOCIETY, Bd. 83, Nr. 7, 1. Januar 2006 (2006-01-01), Seiten 659-662, XP055661876, offenbart ein IMAC-Verfahren zur Aufreinigung von Arachidonsäure (MW=304.5 g/mol) aus fermentativ hergestelltem Einzel-Zell-Pilzöl.

Bei den in dem erfindungsgemäßen Verfahren zu identifizierenden bzw. identifizierten Naturstoffen handelt es sich nicht um Proteine. Unter Proteinen werden im Rahmen des vorliegenden Textes vorzugsweise Substanzen verstanden, die aus mehr als 100 Aminosäuren aufgebaut sind, bevorzugt aus mehr als 50 Aminosäuren, insbesondere aus mehr als 25 Aminosäuren.

Die in dem erfindungsgemäßen Verfahren zu identifizierenden bzw. identifizierten Naturstoffe sind keine Substanzen mit einem Molekulargewicht von 20 kDa oder mehr, sondern weisen die Naturstoffe ein Molekulargewicht von kleiner oder gleich 1000 g/mol auf.

Bevorzugte Lebensmittelextrakte sind Extrakte von Lebensmitteln, die aus verarbeiteten pflanzlichen Produkten erhalten werden, wie beispielsweise geröstete Kaffeebohnen, bei denen viele der darin enthaltenen sensorisch wirksamen Substanzen erst beim Rösten entstehen.

Der in dem erfindungsgemäßen Verfahren in Schritt (a) bereitgestellte und in Schritt (c) eingesetzte naturstoffhaltige Extrakt ist vorzugsweise
- ein wässriger Extrakt (d.h. ein Extrakt, bei dem nur Wasser als Extraktionsmittel eingesetzt wurde), oder
- ein alkoholischer Extrakt mit einem oder mehreren C1-C4-Alkoholen (d.h. ein Extrakt, bei dem nur ein C1-C4-Alkohol oder eine Mischung aus C1-C4-Alkoholen als Extraktionsmittel eingesetzt wurde), dabei vorzugsweise Ethanol, oder
- ein wässrig-alkoholischer Extrakt mit einem oder mehreren C1-C4-Alkoholen (d.h. ein Extrakt, der mittels Extraktion mit einem Gemisch bestehend aus Wasser und C1-C4-Alkohol(en) hergestellt wurde), dabei vorzugsweise ein wässrig-ethanolischer Extrakt.

Die Adsorption in Schritt (c) erfolgt mittels Komplexbildung (Chelatisierung, Komplexierung,) von Naturstoff und den an die stationäre Phase gebundenen Metallionen.

Das in dem erfindungsgemäßen Verfahren in Schritt (d) eingesetzte Lösungsmittel ist ausgewählt aus der Gruppe bestehend aus reinem Wasser, Ethanol, Methanol und deren Mischungen. In Schritt (d) bevorzugt eingesetzte Lösungsmittel sind ausgewählt aus der Gruppe bestehend aus reinem Wasser, vorzugsweise mit ionenfreiem Wasser, und Wasser-Ethanol-Gemischen.

Vorzugsweise wird dabei in Schritt (d) mit einer 5fachen, vorzugsweise mit einer 10fachen, Menge an Lösungsmittel gespült, bezogen auf das Leervolumen der IMAC-Vorrichtung.

In dem erfindungsgemäßen Verfahren wird in Schritt (e) vorzugsweise eine Lösung eines Desorptionsmittels eingesetzt, enthaltend eine oder mehrere Verbindungen aus der Gruppe bestehend aus EDTA, NaEDTA, Na₂EDTA, CaNa₂EDTA, Oxalsäure, Citronensäure, Phytinsäure und Ethylhexansäure.

Bevorzugte Desorptionsmittel in Schritt (d) sind EDTA und EDTA-Salze, wiederum bevorzugt gewählt aus der Gruppe bestehend aus EDTA, NaEDTA und Na₂EDTA. Insbesondere in diesen Fällen sind die erfindungsgemäß identifizierten Naturstoffe weniger stark mit Ni²⁺, Cu²⁺ oder Fe²⁺ - Ionen komplexierende Substanzen als EDTA und EDTA-Salze, was in besonderem Maße gewünscht und vorteilhaft ist (siehe oben).

Die in dem erfindungsgemäßen Verfahren in Schritt (e) eingesetzte Lösung ist vorzugsweise eine wässrige, eine ethanolisch-wässrige oder eine methanolisch-wässrige Lösung eines Desorptionsmittels.

Geeignete Methoden zur Trennung der zur Komplexbildung mit Ni²⁺, Cu²⁺ oder Fe²⁺ - Ionen befähigten Naturstoffe von dem Desorptionsmittel in Schritt (f) sind dem Fachmann bekannt, wie beispielsweise HPLC (Hochleistungs-Flüssigkeitschromatographie). Dabei wird die HPLC - Trennung vorzugsweise unter Verwendung einer C-18 Phase und einer HILIC-Phase (HILIC: Hydrophilic Interaction Chromatography; Hydrophile Interaktionschromatographie; Wässrige Normal-Phasen Chromatographie) durchgeführt.

Die abgetrennten und isolierten, zur Komplexbildung mit Ni²⁺, Cu²⁺ oder Fe²⁺ - Ionen befähigten Naturstoffe werden zuletzt in Schritt (g) identifiziert, d.h. deren Struktur aufgeklärt. Dies geschieht vorzugsweise mittels Massenspektroskopie (MS) und/oder mittels NMR-Spektroskopie.

Hinsichtlich weiterer bevorzugter Ausgestaltungen des erfindungsgemäßen Verfahrens gelten die jeweils oben als bevorzugt bzw. besonders bevorzugt bezeichneten Ausgestaltungen der jeweiligen Verfahrensschritte entsprechend.

In einer besonders bevorzugten Ausführungsform betrifft die vorliegende Erfindung ein Verfahren zur Identifizierung eines zum Verzehr durch den Menschen geeigneten Naturstoffes, insbesondere eines Aroma- oder Geschmackstoffes, der zur Komplexbildung mit Fe²⁺ befähigt ist, gekennzeichnet durch folgende Schritte:
(a) Bereitstellen eines wässrigen, ethanolischen oder wässrig-ethanolischen naturstoffhaltigen Extraktes, wobei der eingesetzte naturstoffhaltige Extrakt ein pflanzlicher Lebensmittelextrakt ist, der durch Extraktion eines oder mehrerer zum Verzehr durch den Menschen geeigneter Pflanzenteile erhalten wurde, ausgewählt aus der Gruppe bestehend aus Stängeln, Blättern, Blüten, Wurzeln, Samen, Früchten,
(b) Bereitstellen einer Vorrichtung, enthaltend eine mit Fe²⁺ - Ionen beladene stationäre Phase, welche zur immobilisierten Metallionen-Affinitäts-Chromatographie (IMAC) geeignet ist (IMAC-Vorrichtung),
(c) Durchleiten des naturstoffhaltigen Extraktes aus Schritt (a) durch eine Vorrichtung aus Schritt (b), so dass eine Adsorption des zur Komplexbildung mit Fe²⁺ - Ionen befähigten Naturstoffes an der stationären Phase der IMAC-Vorrichtung erfolgt,
(d) Spülen der mit dem zur Komplexbildung mit Fe²⁺ - Ionen befähigten Naturstoff beladenen stationären Phase der IMAC-Vorrichtung aus Schritt (c) mit einem Lösungsmittel, ausgewählt aus der Gruppe bestehend aus reinem Wasser und Wasser-Ethanol-Gemischen,
(e) Desorption des Naturstoffes von der stationären Phase der IMAC-Vorrichtung aus Schritt aus Schritt (d) mittels Durchleiten einer Lösung enthaltend EDTA, NaEDTA und/oder Na₂EDTA, durch die IMAC-Vorrichtung aus Schritt (d),
(f) Abtrennung des Naturstoffes vom Desorptionsmittel EDTA, NaEDTA und/oder Na₂EDTA,
(g) Identifizierung des zur Komplexbildung mit Ni²⁺, Cu²⁺ und/oder Fe²⁺ - Ionen befähigten Naturstoffes, vorzugsweise mittels Massen- und/oder NMR-Spektroskopie.

Die in dem erfindungsgemäßen Verfahren jeweils eingesetzten Flüssigkeiten werden vorzugsweise jeweils mit Flussraten im Bereich von 0,5 - 20 ml/min, bevorzugt von 1 - 10 ml/min, über die stationäre Phase geleitet, vorzugsweise gepumpt. Diese Angaben gelten für IMAC-Vorrichtungen, insbesondere IMAC-Säulen, mit einem (Säulen)Volumen von 1 - 20 ml.

Das erfindungsgemäße Verfahren kann mit diversen IMAC-Vorrichtungen durchgeführt werden. IMAC-Materialien sind dem Fachmann bekannt sowie kommerziell erhältlich, beispielsweise als IMAC-Select Affinity Gel (Hersteller: Sigma-Aldrich) oder als IMAC-Säulen ohne Metallionen - Beladung, beispielsweise unter dem Namen IMAC Sepharose 6 Fast Flow (Hersteller: GE Healthcare), z.B. in Form der HiTrap IMAC FF Säulen.

Die Parameter zur Durchführung des erfindungsgemäßen Verfahrens ist in gewissen Grenzen von der jeweils eingesetzten IMAC-Vorrichtung abhängig, insbesondere von der Dimensionierung und dem verwendeten Material der stationären Phase. Der Fachmann kann diese Parameter leicht unter Berücksichtigung der Herstellerangaben und des Herstellerhandbuchs ermitteln und das erfindungsgemäße Verfahren durchführen.

Im Folgenden werden einige bevorzugte Durchführungsarten des erfindungsgemäßen Verfahrens erläutert.

Vorzugsweise wird das erfindungsgemäße Verfahren bei Raumtemperatur (20°C) und unter Berücksichtigung der Angaben im Herstellerhandbuch durchgeführt.

Vor Beladung der IMAC-Vorrichtung mit Metallionen wird vorzugsweise zunächst die stationäre Phase der IMAC-Vorrichtung mit Wasser, vorzugsweise ionenfreiem Wasser, gespült.

Dann wird das feste Adsorbens, d.h. die stationäre Phase, der IMAC-Vorrichtung mit Ni²⁺, Cu²⁺ oder Fe²⁺ - Ionen beladen. Hierzu wird vorzugsweise eine 0,025 - 0,25 M, bevorzugt eine 0,1 M, Metallkationen-Lösung eines Ni²⁺, Cu²⁺ oder Fe²⁺ - Salzes hergestellt und mit der zu beladenden stationären Phase kontaktiert, wobei die Metallkationen von den chelatisierenden Liganden der stationären Phase gebunden werden.

Bevorzugte Ni²⁺, Cu²⁺ oder Fe²⁺ - Salze sind dabei wasserlösliche Salze dieser Metallionen, bevorzugtes Fe²⁺ - Salz ist Fe(NH₄)₂(SO₄)₂ x 6 H₂O.

Die Beladung der stationären Phase mit der Metallkationen-Lösung erfolgt vorzugsweise mit einer Menge an Metallkationen-Lösung von zumindest einem halben Volumenanteil, bezogen auf das Leervolumen der IMAC-Vorrichtung.

Danach wird die mit Ni²⁺, Cu²⁺ oder Fe²⁺ - Ionen beladene stationäre Phase mit Wasser, vorzugsweise mit ionenfreiem Wasser, oder einem ethanolisch-wässrigen Lösungsmedium gespült, um die überschüssigen, ungebundenen Ni²⁺, Cu²⁺ oder Fe²⁺ - Ionen zu entfernen. Dabei wird vorzugsweise mit einer zumindest 5fachen Menge an Wasser oder ethanolisch-wässrigen Lösungsmedium gespült, bezogen auf das Leervolumen der IMAC-Vorrichtung.

Der zu untersuchende naturstoffhaltige Extrakt wird vorzugsweise als verdünnte wässrige Lösung oder als verdünnte, wässrig-ethanolische Lösung eingesetzt.

Der naturstoffhaltige Extrakt wird vorzugsweise vor dessen Durchleiten durch die IMAC-Vorrichtung über einen Membranfilter filtriert, vorzugsweise mit einer Porengröße von 0,45 µm.

Anschließend wird der naturstoffhaltige Extrakt vorzugsweise in der doppelten, vorzugsweise in der dreifachen Menge, bezogen auf das Leervolumen der IMAC-Vorrichtung, auf die IMAC-Vorrichtung gegeben und durch die IMAC-Vorrichtung geleitet.

Beim Durchleiten des naturstoffhaltigen Extraktes durch das mit Metallkationen-beladene Adsorbens werden die zur Komplexbildung mit Ni²⁺, Cu²⁺ oder Fe²⁺ - Ionen befähigten Naturstoffe von der stationären Phase zurückgehalten und verbleiben dort (Adsorption).

Die übrigen, nicht von den Metallkationen der stationären Phase komplexierten Verbindungen des Extraktes werden anschließend durch Spülen mit dem entsprechenden Lösungsmedium entfernt, bevorzugt sind dabei destilliertes Wasser oder ethanolisch-wässrige Lösungsmedien.

Vorzugsweise wird dabei mit einer 10fachen Menge an Lösungsmittel gespült, bezogen auf das Leervolumen der IMAC-Vorrichtung.

Zum Ablösen der zur Komplexbildung mit Ni²⁺, Cu²⁺ oder Fe²⁺ - Ionen befähigten Naturstoffe von der stationären Phase wird das Adsorbens mit einer Lösung enthaltend ein oder mehrere Desorptionsmittel, vorzugsweise einer wässrigen Lösung von EDTA, NaEDTA oder Na₂EDTA, gespült.

Die Konzentration des Desorptionsmittels liegt dabei vorzugsweise im Bereich von 10 - 100 mM.

Vorzugsweise wird dabei mit einer 5fachen, bevorzugt einer 10fachen Menge, einer Lösung enthaltend ein oder mehrere Desorptionsmittel gespült, bezogen auf das Leervolumen der IMAC-Vorrichtung.

Dabei löst das Desorptionsmittel die Bindung zwischen den kovalent an das Adsorbens gebundenen chelatisierenden Liganden und den Metallkationen, in dem es selbst an die Metallkationen bindet.

Somit können die zur Komplexbildung mit Ni²⁺, Cu²⁺ oder Fe²⁺ - Ionen befähigten Naturstoffe aus dem naturstoffhaltigen Extrakt nicht mehr von der stationären Phase zurückgehalten werden und werden bei diesem Spülvorgang ausgespült (Elution).

Die so gewonnene Mischung enthaltend Desorptionsmittel, zur Komplexbildung mit Ni²⁺, Cu²⁺ oder Fe²⁺ - Ionen befähigte Naturstoffe und gegebenenfalls Ni²⁺, Cu²⁺ oder Fe²⁺ - Ionen wird anschließend aufgetrennt und analysiert.

Geeignete Methoden zur Abtrennung der zur Komplexbildung mit Ni²⁺, Cu²⁺ oder Fe²⁺ - Ionen befähigten Naturstoffe und Desorptionsmittel sind beispielsweise HPLC (Hochleistungs-Flüssigkeitschromatographie) unter Verwendung einer C-18 Phase und einer HILIC-Phase (HILIC: Hydrophilic Interaction Chromatography; Hydrophile Interaktionschromatographie; Wässrige Normal-Phasen Chromatographie).

Werden die zur Komplexbildung mit Ni²⁺, Cu²⁺ oder Fe²⁺ - Ionen befähigten Naturstoffe von der C-18 Phase zurückgehalten, erfolgt die Abtrennung des Desorptionsmittels vorzugsweise durch eine Festphasenextraktion (Solid Phase Extraction, SPE) an einer C-18-Phase.

Wenn die zur Komplexbildung mit Ni²⁺, Cu²⁺ oder Fe²⁺ - Ionen befähigten Naturstoffe durch die HILIC-Phase zurückgehalten werden, erfolgt die Abtrennung des Desorptionsmittels vorzugsweise durch eine präparative HPLC (PHPLC) an einer HILIC-Phase.

Die so abgetrennten zur Komplexbildung mit Ni²⁺, Cu²⁺ oder Fe²⁺ - Ionen befähigten Naturstoffe werden dann analysiert und identifiziert, vorzugsweise mittels Massenspektroskopie (z.B. LC-MS) und mittels NMR-Spektroskopie identifiziert.

Die mit dem erfindungsgemäßen Verfahren identifizierten, zur Komplexbildung mit Ni²⁺, Cu²⁺ und/oder Fe²⁺ - Ionen befähigten Naturstoffe können gezielt zur sensorischen Stabilisierung von oxidationsempfindlichen oral konsumierbaren Zubereitungen eingesetzt werden.

Geeignete Kriterien für das Maß der Oxidation sind beispielsweise die Peroxidzahl, die Messung (der Menge) an flüchtigen Aldehyden (insbesondere von n-Hexanal) und die Messung (der Menge) an Malondialdehyd.

Die vorliegende Erfindung betrifft ferner die Verwendung einer mit Ni²⁺, Cu²⁺ und/oder Fe²⁺ - Ionen beladenen stationären Phase, welche zur immobilisierten Metallionen-Affinitäts-Chromatographie (IMAC) geeignet ist, in einem Verfahren nach Anspruch 1.

Eine erfindungsgemäß bevorzugte Verwendung ist dadurch gekennzeichnet, dass der Naturstoff ein zum Verzehr durch den Menschen geeigneter Naturstoff ist, und vorzugsweise ausgewählt ist aus der Gruppe bestehend aus Aromastoffen und Geschmackstoffen.

Das erfindungsgemäße Verfahren und eine zum Durchführen des Verfahrens besonders geeignete Vorrichtung wird nachfolgend anhand der Ausführungsbeispiele näher beschrieben, ohne dass hierdurch der Schutzbereich gegenüber den Patentansprüchen eingeschränkt werden soll.

### Beispiel 1: Methode - Durchführung der Immobilisierten Metallionen-Affinitäts-Chromatographie (IMAC)

Alle Versuche wurden bei Raumtemperatur und unter Berücksichtigung der Angaben im Herstellerhandbuch der IMAC-Säule durchgeführt.

Als stationäre Phase (festes Adsorbens) für das IMAC-Verfahren wurde IMAC Sepharose^{®} 6 Fast Flow Material der Firma GE Healthcare (General Electric Healthcare) verwendet.

Bei dieser Matrix handelt es sich um eine kugelförmige, kreuzvernetzte (cross-linked) 6 % Agarose mit einer durchschnittlichen Partikelgröße von 90 µm. An diese Matrix sind chelatisierende Liganden kovalent gebunden.

Es wurden IMAC-Säulen mit 1 ml Säulenvolumen und mit 20 ml Säulenvolumen verwendet. Die Durchflussmengen wurden dabei so eingestellt, dass die Flussraten bei 1,3 ml/min lagen im Falle der IMAC-Säulen mit 1 ml Säulenvolumen (bei max. p von 0,30 MPa) und bei 6,3 ml/ml im Falle der IMAC-Säulen mit 20 ml Säulenvolumen (bei max. p von 0,15 MPa).

Nach dem Spülen der stationären Phase mit dem fünffachen Säulenvolumen an destilliertem Wasser, wurde die stationäre Phase mit Fe²⁺ - Ionen beladen.

Es wurde eine wässrige 0,1 M Fe²⁺-Lösung aus Fe(NH₄)₂(SO₄)₂ × 6 H₂O und destilliertem Wasser durch das Adsorbens geleitet und dadurch das Adsorbens mit Fe²⁺-Ionen beladen.

Danach wurde die mit Fe²⁺-Ionen beladene stationäre Phase erneut mit dem fünffachen Säulenvolumen an destilliertem Wasser gespült, um die überschüssigen / ungebundenen Metallkationen zu entfernen.

Als Lösungsmedien für den naturstoffhaltigen Extrakt werden vorzugsweise destilliertes Wasser oder ethanolisch-wässrige Lösungsmedien verwendet, je nach Löslichkeit des Extraktes.

Der jeweilige zu untersuchende naturstoffhaltige Extrakt (im Folgenden auch Naturstoffextrakt) wurde als verdünnte, wässrige Lösung oder als verdünnte, wässrig-ethanolische Lösung eingesetzt.

Alle Versuche wurden mit membranfiltrierten (0,45 µm) Naturstoffextrakten durchgeführt.

Dieser Naturstoffextrakt wurde anschließend durch das mit Metallkationen-beladene Adsorbens geleitet und die Verbindungen mit den Fe²⁺-Ionen-komplexierenden Eigenschaften wurden von der stationären Phase zurückgehalten (Adsorption).

Die übrigen, nicht von den Fe²⁺-Ionen der stationären Phase komplexierten Verbindungen des Naturstoffextraktes wurden anschließend durch Spülen mit dem zehnfachen Säulenvolumen an destilliertem Wasser von der IMAC-Säule gespült.

Zum Ablösen der zur Komplexbildung mit Fe²⁺ - Ionen befähigten Naturstoffe von der stationären Phase wurde das Adsorbens mit dem zehnfachen Säulenvolumen einer wässrigen 50 mM EDTA-Lösung (hergestellt aus Na₂EDTA x 2 H₂O und destilliertem Wasser) von der IMAC-Säule gespült.

Die so gewonnene Mischung aus EDTA, Fe²⁺ und der zur Komplexbildung mit Fe²⁺ - Ionen befähigten Naturstoffe wurde mittels HPLC (Hochleistungs-Flüssigkeitschromatographie) unter Verwendung eine C-18 Phase und einer HILIC-Phase (HILIC: Hydrophilic Interaction Chromatography; Hydrophile Interaktionschromatographie; Wässrige Normal-Phasen Chromatographie) analysiert.

Wurden die komplexierenden Verbindungen von der C-18 Phase zurückgehalten, erfolgte die Abtrennung des EDTA durch eine Festphasenextraktion (Solid Phase Extraction, SPE) an einer C-18-Phase. Wenn die Verbindungen durch die HILIC-Phase zurückgehalten wurden, erfolgte die EDTA-Abtrennung durch eine präparative HPLC an einer HILIC-Phase. Die Naturstoffe wurden danach mittels LC-MS analysiert und deren Struktur mittels NMR-Spektroskopie identifiziert.

### Beispiel 2: Grüntee-Extrakt

Die Durchführung erfolgte nach der in Beispiel 1 beschriebenen Methode. Für die Durchführung des Verfahrens mit Grüntee-Extrakt wurde der Extrakt als verdünnte wässrige Lösung von grünem Tee eingesetzt (10 mg/ml) (10 mg Extrakt in 1 ml Wasser gelöst). Die Beladung des Adsorbens erfolgte mit einer wässrigen 0,1 M Fe²⁺-Lösung aus Fe(NH₄)₂(SO₄)₂ × 6 H₂O und Wasser. Nach der Beladung wurde mit destilliertem Wasser gespült und danach die wässrige Lösung des Grüner-Tee-Extraktes durch das mit Fe²⁺-Ionen beladene Adsorbens geleitet. Nach dem Spülen mit Wasser wurden die komplexierenden Naturstoffe mit 50 mM EDTA-Lösung eluiert. Die anschließende HPLC Analyse zeigte, dass die enthaltenen Verbindungen an einer C-18-Phase zurückgehalten werden, deshalb ist eine SPE an einer C-18-Phase zur Abtrennung des EDTA durchgeführt worden. Bei der anschließenden Analyse konnte Epigallocatechingallat als Fe²⁺-komplexierender Naturstoff identifiziert werden.

### Beispiel 3: Malzextrakt

Die Durchführung erfolgte nach der in Beispiel 1 beschriebenen Methode. Für die Durchführung des Verfahrens mit Malzextrakt wurde der Extrakt als verdünnte wässrige Lösung eingesetzt (10 mg/ml). Die Beladung des Adsorbens erfolgte wie in Beispiel 2 wiederum mit Fe²⁺-Ionen. Nach der Beladung wurde mit destilliertem Wasser gespült und danach die wässrige Lösung des Malzextraktes durch das mit Fe²⁺-Ionen beladene Material geleitet. Nach dem Spülen mit Wasser wurden die komplexierenden Naturstoffe mit 50 mM EDTA-Lösung eluiert. Die anschließende HPLC Analyse zeigte, dass die enthaltenen Verbindungen an einer HILIC-Phase zurückgehalten werden. Um im Vorfeld einen Großteil des EDTA abzutrennen, wurde Ethanol zu dieser Lösung gegeben, was eine Ausfällung des EDTA bewirkte, so dass das EDTA abfiltriert werden konnte. Danach wurde eine Abtrennung des restlichen EDTA durch eine PHPLC (präparative HPLC) an der HILIC-Phase unter Verwendung von Wasser und Acetonitril durchgeführt.

### Beispiel 4: Kaffee-Extrakt

Die Durchführung erfolgte nach der in Beispiel 1 beschriebenen Methode. Für die Durchführung des Verfahrens mit Kaffee-Extrakt wurde der Extrakt als verdünnte wässrige Lösung eingesetzt, hergestellt aus 5 g Kaffeepulver und 25 g kochendem Wasser. Die Beladung des Adsorbens erfolgte wie in Beispiel 2 wiederum mit Fe²⁺-Ionen. Nach der Beladung wurde mit destilliertem Wasser gespült und danach die wässrige Lösung des Kaffeeextraktes durch das mit Fe²⁺-Ionen beladene Adsorbens geleitet. Nach dem Spülen mit Wasser wurden die komplexierenden Verbindungen mit 50 mM wässriger EDTA-Lösung eluiert. Die anschließende HPLC Analyse zeigte, dass die enthaltenen Verbindungen an einer HILIC-Phase zurückgehalten werden. Um im Vorfeld einen Großteil des EDTA abzutrennen, ist Ethanol zu dieser Lösung gegeben worden, was eine Ausfällung des EDTA bewirkte, so dass das EDTA abfiltriert werden konnte. Danach wurde eine Abtrennung des restlichen EDTA durch die PHPLC (präparative HPLC) an der HILIC-Phase unter Verwendung von Wasser und Acetonitril durchgeführt.

## Patentansprüche

1. Verfahren zur Identifizierung eines Naturstoffes, der zur Komplexbildung mit Ni²⁺-, Cu²⁺- und/oder Fe²⁺- Ionen befähigt ist, **gekennzeichnet durch** folgende Schritte:
(a) Bereitstellen eines wässrigen, alkoholischen oder wässrig-alkoholischen naturstoffhaltigen Extraktes, wobei der eingesetzte naturstoffhaltige Extrakt ein pflanzlicher Lebensmittelextrakt ist, der durch Extraktion eines oder mehrerer zum Verzehr durch den Menschen geeigneter Pflanzenteile erhalten wurde, ausgewählt aus der Gruppe bestehend aus Stängeln, Blättern, Blüten, Wurzeln, Samen, Früchten,
(b) Bereitstellen einer Vorrichtung, enthaltend eine mit Ni²⁺, Cu²⁺ und/oder Fe²⁺ - Ionen beladene stationäre Phase, welche zur immobilisierten Metallionen-Affinitäts-Chromatographie (IMAC) geeignet ist (IMAC-Vorrichtung),
(c) Durchleiten des naturstoffhaltigen Extraktes aus Schritt (a) durch eine Vorrichtung aus Schritt (b), so dass eine Adsorption des zur Komplexbildung mit Ni²⁺, Cu²⁺ und/oder Fe²⁺ - Ionen befähigten Naturstoffes an der stationären Phase der IMAC-Vorrichtung erfolgt,
(d) gegebenenfalls Spülen der mit dem zur Komplexbildung mit Ni²⁺, Cu²⁺ und/oder Fe²⁺ - Ionen befähigten Naturstoff beladenen stationären Phase der IMAC-Vorrichtung aus Schritt (c) mit einem Lösungsmittel ausgewählt aus der Gruppe bestehend aus reinem Wasser, vorzugsweise ionenfreiem Wasser, und Wasser-Ethanol-Gemischen,
(e) Desorption des Naturstoffes von der stationären Phase der IMAC-Vorrichtung aus Schritt (c) oder gegebenenfalls aus Schritt (d), bevorzugt mittels Durchleiten einer Lösung enthaltend ein oder mehrere Desorptionsmittel durch die IMAC-Vorrichtung aus Schritt (c) oder gegebenenfalls aus Schritt (d),
(f) gegebenenfalls Abtrennung des Naturstoffes vom Desorptionsmittel,
(g) Identifizierung des zur Komplexbildung mit Ni²⁺, Cu²⁺ und/oder Fe²⁺ - Ionen befähigten Naturstoffes,
wobei der Naturstoff kein Protein ist und ein Molekulargewicht von kleiner oder gleich 1000 g/mol aufweist.

2. Verfahren nach Anspruch 1, **dadurch gekennzeichnet, dass** der in Schritt (a) bereitgestellte und in Schritt (c) eingesetzte naturstoffhaltige Extrakt
- ein wässriger Extrakt ist, oder
- ein alkoholischer Extrakt mit einem oder mehreren C1-C4-Alkoholen, dabei vorzugsweise Ethanol, oder
- ein wässrig-alkoholischer Extrakt mit einem oder mehreren C1-C4-Alkoholen, dabei vorzugsweise ein wässrig-ethanolischer Extrakt, ist.

3. Verfahren nach einem der Ansprüche 1 oder 2, **dadurch gekennzeichnet dass** in Schritt (e) eine Lösung eines Desorptionsmittels eingesetzt wird, enthaltend eine oder mehrere Verbindungen aus der Gruppe bestehend aus EDTA, NaEDTA, Na₂EDTA, CaNa₂EDTA, Oxalsäure, Citronensäure, Phytinsäure und Ethylhexansäure, vorzugsweise aus der Gruppe bestehend aus EDTA, NaEDTA und Na₂EDTA.

4. Verfahren nach einem der Ansprüche 1 bis 3, **dadurch gekennzeichnet, dass** die in Schritt (e) eingesetzte Lösung eine wässrige, eine ethanolisch-wässrige oder eine methanolisch-wässrige Lösung eines Desorptionsmittels ist.

5. Verfahren nach einem der Ansprüche 1 bis 4, **dadurch gekennzeichnet, dass** in Schritt (f) die Abtrennung des Naturstoffes vom Desorptionsmittel erfolgt mittels Extraktion, dabei vorzugsweise mittels SPE (Solid Phase Extraction; Festphasenextraktion), Flüssig/Flüssig-Verteilung, Ausfällung oder mittels Chromatographie, dabei vorzugsweise Gelchromatographie, HPLC oder HILIC (Hydrophilic Interaction Chromatography; Hydrophile Interaktionschromatographie; Wässrige Normal-Phasen Chromatographie).

6. Verwendung einer mit Ni^{2+ -}, Cu^{2+ -} und/oder Fe²⁺ - Ionen beladenen stationären Phase, welche zur immobilisierten Metallionen-Affinitäts-Chromatographie (IMAC) geeignet ist, in einem Verfahren gemäß einem der Ansprüche 1 bis 5.

## Claims

1. A method for identifying a natural substance capable of complex formation with Ni²⁺, Cu²⁺ and/or Fe²⁺ ions, **characterized by** the following steps
(a) providing an aqueous, alcoholic or aqueous-alcoholic extract containing a natural substance, wherein the used extract containing a natural substrate is a plant-based food extract obtained by extraction of one or more parts of a plant, which is/are suitable for human consumption, selected from the group consisting of stems, leaves, flowers, roots, seeds, fruits,
(b) providing a device containing a stationary phase loaded with Ni²⁺, Cu²⁺ and/or Fe²⁺ ions, which is suitable for immobilized metal ion affinity chromatography (IMAC) (IMAC device),
(c) passing the extract containing a natural substrate of step (a) through a device of step (b), such that an adsorption of the natural substance capable of complex formation with Ni²⁺, Cu²⁺ and/or Fe²⁺ ions onto the stationary phase of the IMAC device is obtained,
(d) optionally, rinsing the stationary phase loaded with the natural substance capable of complex formation with Ni²⁺, Cu²⁺ and/or Fe²⁺ ions of the IMAC device of step (c) with a solvent selected from the group consisting of pure water, preferably ion-free water, and water-ethanol mixtures,
(e) desorption of the natural substance from the stationary phase of the IMAC device of step (c) or step (d), preferably by passing a solution containing one or more desorbents through the IMAC device of step (c) or of step (d),
(f) optionally, separation of the natural substance from the desorbent,
(g) identification of the natural substance capable of complex formation with Ni²⁺, Cu²⁺ and/or Fe²⁺ ions, wherein the natural substance is not a protein and has a molecular weight of less than or equal to 1000 g/mol.

2. The method according to claim 1, **characterized in that** the extract containing a natural substance provided in step (a) and used in step (c) is
- an aqueous extract, or
- an alcoholic extract with one or more C1-C4-alcohols, preferably ethanol, or
- an aqueous-alcoholic extract with one or more C1-C4-alcohols, preferably an aqueous ethanolic extract.

3. The method according to any one of claims 1 or 2, **characterized in that** in step (e) a solution of a desorbent is used, containing one or more compounds selected from the group consisting of EDTA, NaEDTA, Na₂EDTA, CaNa₂EDTA, oxalic acid, citric acid, phytic acid, and ethylhexanoic acid, preferably from the group consisting of EDTA, NaEDTA and Na₂EDTA.

4. The method according to any one of claims 1 to 3, **characterized in that** the solution used in step (e) is an aqueous, an ethanolic-aqueous or a methanolic-aqueous solution of a desorbent.

5. The method according to any one of claims 1 to 4, **characterized in that** in step (f) the separation of the natural substance from the desorbent is obtained by extraction, preferably by SPE (Solid Phase Extraction), liquid/liquid partitioning, precipitation or by chromatography, preferably by gel chromatography, HPLC or HILIC (Hydrophilic Interaction Chromatography; Aqueous Normal Phase chromatography).

6. A use of a stationary phase loaded with Ni²⁺, Cu²⁺ and/or Fe²⁺ ions, which is suitable for immobilized metal ion affinity chromatography (IMAC) in a method according to any one of claims 1 to 5.

## Revendications

1. Procédé d'identification d'une substance naturelle qui est capable de former des complexes avec des ions Ni²⁺, Cu²⁺ et/ou Fe²⁺ , **caractérisé par** les étapes suivantes consistant à:
(a) fournir un extrait aqueux, alcoolique ou hydro-alcoolique contenant des substances naturelles, dans lequel l'extrait contenant des substances naturelles utilisé est un extrait alimentaire végétal qui a été obtenu par extraction d'une ou de plusieurs parties végétales propres à la consommation humaine, choisies dans le groupe constitué par les tiges, les feuilles, les fleurs, les racines, les graines, les fruits,
(b) fournir un dispositif contenant une phase stationnaire chargée en ions Ni²⁺, Cu²⁺ et/ou Fe²⁺ qui est adaptée à la chromatographie d'affinité sur ions métalliques immobilisés (IMAC) (dispositif IMAC),
(c) faire passer l'extrait contenant des substances naturelles de l'étape (a) à travers un dispositif de l'étape (b) de sorte qu'il a lieu une adsorption de la substance naturelle capable de former des complexes avec des ions Ni²⁺, Cu²⁺ et/ou Fe²⁺ sur la phase stationnaire du dispositif IMAC,
(d) rincer le cas échéant la phase stationnaire du dispositif IMAC de l'étape (c), qui est chargée de la substance naturelle capable de former des complexes avec des ions Ni²⁺, Cu²⁺ et/ou Fe²⁺, avec un solvant choisi dans le groupe constitué par l'eau pure, de préférence l'eau déionisée, et les mélanges eau-éthanol,
(e) désorber la substance naturelle de la phase stationnaire du dispositif IMAC de l'étape (c) ou le cas échéant de l'étape (d), de préférence en faisant passer une solution contenant un ou plusieurs agents de désorption à travers le dispositif IMAC de l'étape (c) ou le cas échéant de l'étape (d),
(f) séparer le cas échéant la substance naturelle de l'agent de désorption,
(g) identifier la substance naturelle capable de former des complexes avec des ions Ni²⁺, Cu²⁺ et/ou Fe²⁺,
dans lequel la substance naturelle n'est pas une protéine et présente un poids moléculaire inférieur ou égal à 1000 g/mol.

2. Procédé selon la revendication 1, **caractérisé par le fait que** l'extrait contenant des substances naturelles qui est fourni à l'étape (a) et utilisé à l'étape (c)
- est un extrait aqueux, ou
- un extrait alcoolique avec un ou plusieurs alcools en C1-C4, de préférence l'éthanol, ou
- un extrait hydro-alcoolique avec un ou plusieurs alcools en C1-C4, de préférence un extrait hydro-éthanolique.

3. Procédé selon l'une quelconque des revendications 1 ou 2, **caractérisé par le fait que**, à l'étape (e), on utilise une solution d'un agent de désorption contenant un ou plusieurs composés du groupe constitué par l'EDTA, le NaEDTA, le Na₂EDTA, le CaNa₂EDTA, l'acide oxalique, l'acide citrique, l'acide phytique et l'acide éthylhexa-noïque, de préférence du groupe constitué par l'EDTA, le NaEDTA et le Na₂EDTA.

4. Procédé selon l'une quelconque des revendications 1 à 3, **caractérisé par le fait que** la solution utilisée à l'étape (e) est une solution aqueuse, éthanolo-aqueuse ou méthanolo-aqueuse d'un agent de désorption.

5. Procédé selon l'une quelconque des revendications 1 à 4, **caractérisé par le fait que**, à l'étape (f), la substance naturelle est séparée de l'agent de désorption par extraction, de préférence au moyen de SPE (Solid Phase Extraction; extraction en phase solide), de distribution liquide/liquide, de précipitation ou au moyen de chromatographie, de préférence par chromatographie sur gel, HPLC ou HILIC (Hydrophilic Interaction Chromatography, chromatographie à interaction hydrophile; chromatographie en phase normale aqueuse).

6. Utilisation d'une phase stationnaire chargée en ions Ni²⁺, Cu²⁺ et/ou Fe²⁺ qui est adaptée à la chromatographie d'affinité sur ions métalliques immobilisés (IMAC) dans un procédé selon l'une quelconque des revendications 1 à 5.
